# EUROPEAN PATENT APPLICATION

(11) **EP 1 314 423 A1**
(43) Date of publication of application: **28.05.2003**
(21) Application number: 01961177.1
(22) Date of filing: 29.08.2001
(51) Int. Cl.: A61K 31/00, A61K 31/22, A61K 31/366, A61K 31/44, A61K 31/40, A61K 31/505, A61K 31/47, A61K 45/06, A61P 9/10, A61P 3/06, A61P 43/00

(54) **MEDICINAL COMPOSITIONS**

(30) Priority: 01.09.2000 JP 2000265082
(71) Applicant: Sankyo Company, Limited, Tokyo 103-8426 (JP)
(72) Inventor: KOHAMA, Takafumi, Shinagawa-ku Tokyo 140-8710 (JP); INABA, Toshimori, Shinagawa-ku Tokyo 140-8710 (JP)
(74) Representative: Gibson, Christian John Robert
(86) International application number: JP0107438
(87) International publication number: WO02020009

(57) **Abstract**

The present invention provides a pharmaceutical composition for administering simultaneously, separately or sequentially N-(1-octyl-5-carboxymethyl-4,6-dimethylindolin-7-yl)-2,2-dimethylpropanamide or a pharmacologically acceptable salt thereof, and a HMG-CoA reductase inhibitor.

## Description

### Technical field of the invention

The present invention relates to a pharmaceutical composition for administering simultaneously, separately or sequentially N-(1-octyl-5-carboxymethyl-4,6-dimethylindolin-7-yl)-2,2-dimethylpropanamide or its pharmacologically acceptable salt, and a 3-hydroxy-3-methylglutaryl CoA (hereinafter abbreviated as HMG-CoA) reductase inhibitor.

The present invention also relates to a method for the prevention or treatment of atherosclerosis or xanthoma (particularly atherosclerosis), which comprises administering simultaneously, separately or sequentially an effective amount of N-(1-octyl-5-carboxymethyl-4,6-dimethylindolin-7-yl)-2,2-dimethylpropanamide or a pharmacologically acceptable salt thereof, and an HMG-CoA reductase inhibitor to a warm-blooded animal (particularly a human).

### Background of the invention

The incidence of atherosclerosis is continuing to increase accompanying the introduction of a Western diet and the growing proportion of elderly people in the population. Since atherosclerosis is the primary cause of myocardial infarction, cerebral infarction, cerebral apoplexy and so forth, there is a need for its effective prevention and treatment. In addition to hyperlipemia (and particularly hypercholesterolemia), risk factors for atherosclerosis include hypertension, and abnormalities in carbohydrate metabolism based on insulin resistance. There are many cases in which these risk factors cause complications (syndrome X), and their etiology is considered to be mutually interrelated [Diabetes. 37, 1595 (1988)].

Attempts have been made in the past to inhibit each of the risk factors of hyperlipemia, hypertension and insulin resistance for the purpose of preventing or treating atherosclerosis. However, although HMG-CoA reductase inhibitors like pravastatin reduce hyperlipemia, and as a result, demonstrate effectiveness in inhibiting the onset of atherosclerosis, it is known that the effectiveness of a single medicament is not considered to be adequate for patients with serious hyperlipemia or atherosclerosis [Biochim. Biophys. Acta, 960, 294 (1988)]. Thus, a medicament and a therapeutic method having a new effect are required.

A combination of two or more kinds of medicaments having a lipid lowering action is known to be effective in treating hyperlipemia and atherosclerosis [The Washington Manual of Medical Therapeutics, 29th Edition, by Department of Medicine, Washington University School of Medicine (1998)]. In addition, the efficacy of combination of lipid lowering agents having new mechanisms of action with an existing medicament has also been pointed out [Diabete & Metabolisme (Paris) . 21, 139 (1995)]. For example, a pharmaceutical composition of a combination of 2,6-bis(1-methylethyl)phenyl [[2,4,6-tris(1 -methylethyl)phenyl]acetyl] sulfamate with atorvastatin is specifically disclosed in WO 97/16184.

However, many unknown aspects still remain with respect to which combinations of medicaments and HMG-CoA reductase inhibitors make it possible to obtain truly effective and safe lipid lowering agents or prophylactic or therapeutic agents for atherosclerosis. Since some medicaments have potent toxicity (for example, Drugs of the Future, 25, 171 (2000)), avoiding that toxicity is considered to be important in terms of concomitant therapy with multiple drugs. Thus, the selection of drug combinations is an important task.

### Disclosure of the invention

As a result of research in consideration of the importance of prevention and treatment of atherosclerosis, the inventors of the present invention found that a combination of N-(1-octyl-5-carboxymethyl-4,6-dimethylindolin-7-yl)-2,2-dimethylpropanamide or a pharmacologically acceptable salt thereof and an HMG-CoA reductase inhibitor is useful as a prophylactic or therapeutic agent (particularly a therapeutic agent) for atherosclerosis or xanthoma (particularly atherosclerosis). This combination of drugs has been found to exhibit a lipid lowering action, inhibitory effects on the progress of atherosclerosis in the aorta, inhibitory effects on the onset of xanthoma occurring in limb joints and low toxicity.

The present invention provides a pharmaceutical composition (particularly a prophylactic or therapeutic agent for atherosclerosis or xanthoma) for administering simultaneously, separately or sequentially N-(1-octyl-5-carboxymethyl-4,6-dimethylindolin-7-yl)-2,2-dimethylpropanamide or its pharmacologically acceptable salt, and a HMG-COA reductase inhibitor.

The present invention also provides a method for the prevention or treatment of atherosclerosis or xanthoma, which comprises administering simultaneously, separately or sequentially an effective amount of N-(1-octyl-5-carboxymethyl-4,6-dimethylindolin-7-yl)-2,2-dimethylpropanamide or a pharmacologically acceptable salt thereof, and an HMG-CoA reductase inhibitor to a warm-blooded animal (particularly a human).

The active ingredients of the pharmaceutical composition of the present invention are N-(1-octyl-5-carboxymethyl-4,6-dimethylindolin-7-yl)-2,2-dimethylpropanamide or a pharmacologically acceptable salt thereof, and an HMG-CoA reductase inhibitor.

The N-(1-octyl-5-carboxymethyl-4,6-dimethylindolin-7-yl)-2,2-dimethylpropanamide of the present invention is a compound that is described in WO 97/12860 (EP 0866059) and the corresponding USP 6,063,806 (see Example 4) and has the following structural formula:

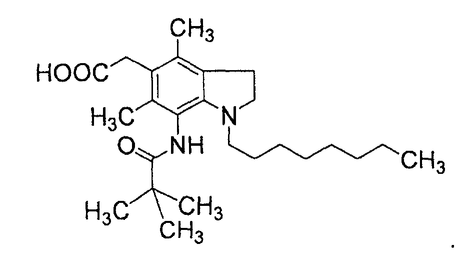

An HMG-CoA reductase inhibitor, which is one of the active ingredients of the pharmaceutical composition of the present invention is inherently used as a therapeutic agent for hyperlipemia, and includes all naturally-occurring substances of microbial origin, semi-synthetic substances derived from them and totally synthetic substances, examples of which include statin compounds such as (+)-(3R,5R)-3,5-dihydroxy-7-[(1S,2S,6S,8S,8aR)-6-hydroxy-2-methyl-8-[(S) -2-methylbutyryloxy]-1,2,6,7,8,8a-hexahydro-1-napthyl]heptanoic acid (hereinafter referred to as pravastatin) described in Japanese Patent Application (Kokai) No. Sho 57-2240 (USP 4,346,227), (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl (S)-2-methylbutyrate (hereinafter referred to as lovastatin) described in Japanese Patent Application (Kokai) No. Sho 57-163374 (USP 4,231,938), (+)-(1S,3R,75,8S,8aR)-1,2,3,7,8,8a-hexahydro-3,7-dimethyl-8-[2-[(2R, 4R)-tetrahydro-4-hydroxy-6-oxo-2H-pyran-2-yl]ethy1]-1-naphthyl 2, 2-dimethylbutyrate (hereinafter referred to as simvastatin) described in Japanese Patent Application (Kokai) No. Sho 56-122375 (USP 4,444,784), (±)(3R*,5S*,6E)- 7-[3-(4-fluorophenyl)-1-(1-methylethyl)-1H-indol-2-yl]-3,5-dihydroxy-6-heptenoic acid (hereinafter referred to as fluvastatin) described in Japanese Patent Application (Kohyo) No. Sho 60-500015 (USP 4,739,073), (3R,5S,6E)-7-[4-(4-fluorophenyl)-2,6-di-(1-methylethyl)-5-methoxymethylpyridin-3-yl]-3, 5-dihydroxy-6-heptenoic acid (hereinafter referred to as rivastatin) described in Japanese Patent Application (Kokai) No. Hei 1-216974 (USP 5,006,530), (3R, 5S) -7-[2-(4-fluorophenyl)-5-(1-methylethyl)-3-phenyl-4-phenylaminocarbonyl-1H-pyroll-1-yl]-3,5-dihydroxyheptanoic acid (hereinafter referred to as atorvastatin) described in Japanese Patent Application (Kokai) No. Hei 3-58967 (USP 5,273,995), (E)-3,5-dihydroxy-7-[4'-(4"-fluorophenyl)-2'-cyclopropylquinoline-3'-yl]-6-heptenoic acid (hereinafter referred to as pitavastatin) which is described in Japanese Patent Application (Kokai) Hei 1-279866 (USP 5,854,259 and USP 5,856,336) or (+)-(3R,5S)-7-[4-(4-fluorophenyl)-6-isopropyl-2-(N-methyl-N-methanesulfonylamino)pyrimidin-5-yl]-3,5-dihydroxy-6(E)-heptenoic acid (hereinafter referred to as rosuvastatin) described in Japanese Patent Application (Kokai) No. Hei 5-178841 (USP 5,260,440). The U.S. patents referenced with respect to each HMG-CoA reductase inhibitor are incorporated herein by reference for their disclosure of the named HMG-CoA reductase inhibitor as well as other HMG-CoA reductase inhibitors, useful in the invention combination (including methods of making the inhibitors) as described herein. Preferred examples include pravastatin, lovastatin, simvastatin, fluvastatin, rivastatin, atorvastatin, rosuvastatin and pitavastatin; more preferred examples include pravastatin, lovastatin, simvastatin, fluvastatin, atorvastatin, rosuvastatin and pitavastatin. Still more preferred examples include pravastatin, atorvastatin and pitavastatin, even more preferred examples include pravastatin and atorvastatin and a particularly preferred example is pravastatin.

Planar structural formulae of typical HMG-CoA reductase inhibitors are shown below.

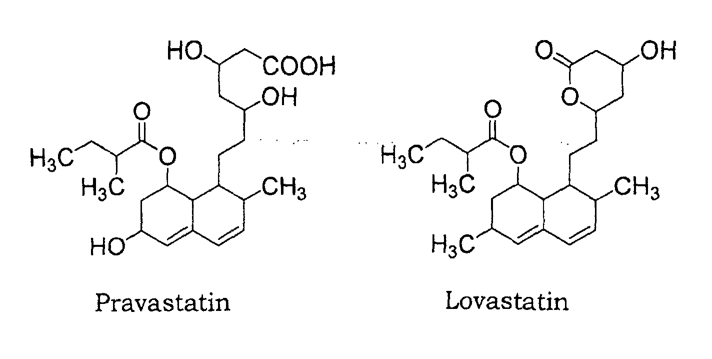

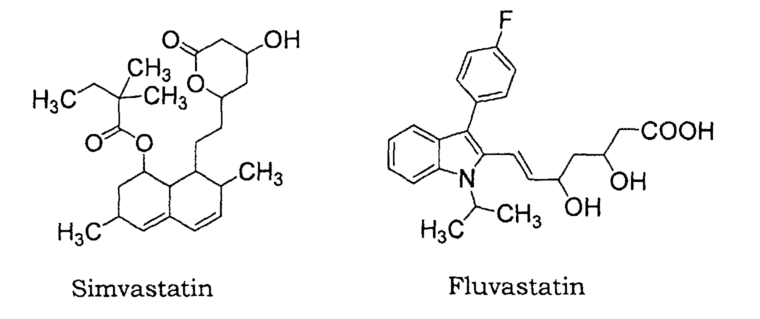

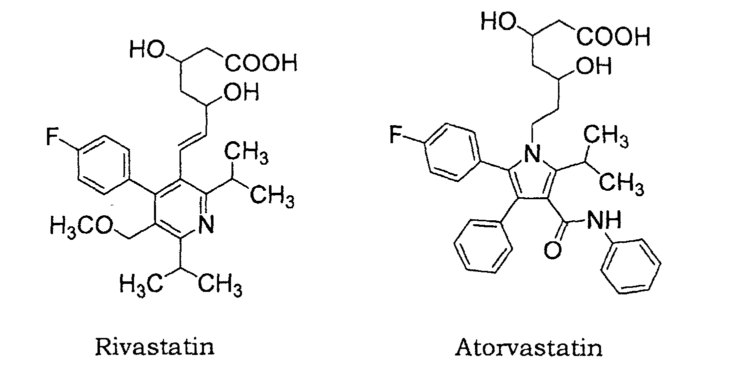

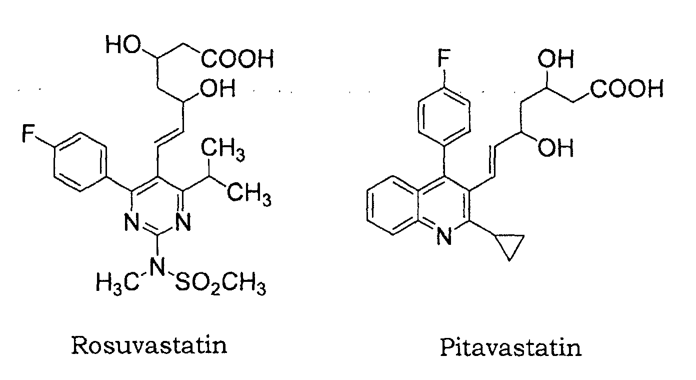

The N-(1-octyl-5-carboxymethyl-4,6-dimethylindolin-7-yl)-2,2-dimethylpropanamide and/or the HMG-CoA reductase inhibitor, which are the active ingredients of the pharmaceutical composition of the present invention, can be converted to a salt in accordance with conventional methods as desired. For example, a salt can be obtained by treating each active ingredient at room temperature for 5 to 30 minutes with the corresponding acid in a solvent (e.g., an ether, ester or alcohol, preferably an ether) followed by either filtering off the precipitated crystals or distilling off the solvent under reduced pressure. Examples of such salts include mineral acid salts such as hydrofluorides, hydrochlorides, hydrobromides, hydroiodides, nitrates, perchlorates, sulfates or phosphates; sulfonates such as methanesulfonates, trifluoromethanesulfonates, ethanesulfonates, benzenesulfonates or p-toluenesulfonates; carboxylates such as fumarates, succinates, citrates, tartrates, oxalates or maleates; or amino acid salts such as glutamates or aspartates.

In addition, the N-(1-octyl-5-carboxymethyl-4,6-dimethylindolin-7-yl)-2,2-dimethylpropanamide and/or the HMG-CoA reductase inhibitor, which are the active ingredients of the pharmaceutical composition of the present invention, can be converted to its respective pharmacologically acceptable salt by treating each active ingredient with a base in accordance with conventional methods as desired. For example, pharmacologically acceptable salts can be obtained by treating each active ingredient at room temperature for 5 to 30 minutes with the corresponding base in a solvent (e.g., an ether, ester or alcohol, preferably an alcohol), followed by either filtering off the precipitated crystals or distilling off the solvent under reduced pressure. Examples of such salts include inorganic salts, for example, alkali metal salts such as sodium salts, potassium salts and lithium salts, alkaline earth metal salts such as calcium salts and magnesium salts, metal salts such as aluminum salts, iron salts, zinc salts, copper salts, nickel salts and cobalt salts; ammonium salts; and organic salts, for example amine salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts, tris(hydroxymethyl)aminomethane salts and the like, while preferred examples include alkali metal salts (particularly sodium salts or calcium salts).

It should be noted that pravastatin, lovastatin, simvastatin, fluvastatin, rivastatin, atorvastatin or pitavastatin includes its lactone ring closure form, or a pharmacologically acceptable salt (preferably a sodium salt or calcium salt) of the HMG-CoA reductase inhibitor, which is an active ingredient of the pharmaceutical composition of the present invention.

For the N-(1-octyl-5-carboxymethyl-4,6-dimethylindolin-7-yl)-2,2-dimethylpropanamide or a pharmacologically acceptable salt thereof, and the HMG-CoA reductase inhibitor, which are the active ingredients of the pharmaceutical composition of the present invention, each geometrical isomer, or stereoisomer in the case of containing an asymmetrical carbon, or their mixtures are also included in the present invention.

The N-(1-octyl-5-carboxymethyl-4,6-dimethylindolin-7-yl)-2,2-dimethylpropanamide or a pharmacologically acceptable salt thereof and the HMG-CoA reductase inhibitor, which are the active ingredients of the pharmaceutical composition of the present invention, can exist as their respective hydrates; each of those hydrates or their mixtures are also included in the present invention.

There are no particular restrictions on the term "administering simultaneously" in the present invention provided it refers to an administration regimen that enables administration to be performed at nearly the same time. The active ingredients are preferably administered in the form of a single composition.

Similarly, there are no particular restrictions on the phrase "administering separately or sequentially" in the present invention provided it refers to an administration regimen that enables administration to be performed separately or sequentially, and it refers to, for example, first administering N-(1-octyl-5-carboxymethyl-4,6-dimethylindolin-7-yl)-2,2-dimethylpropanamide or a pharmacologically acceptable salt thereof followed by administration of an HMG-CoA reductase inhibitor at an appropriate interval, or first administering an HMG-CoA reductase inhibitor followed by administration of N-(1-octyl-5-carboxymethyl-4,6-dimethylindolin-7-yl)-2,2-dimethylpropanamide or a pharmacologically acceptable salt thereof at an appropriate interval.

The pharmaceutical composition of the present invention preferably includes:
(1) a pharmaceutical composition in which the HMG-CoA reductase inhibitor is pravastatin, lovastatin, simvastatin, fluvastatin, rivastatin, atorvastatin, rosuvastatin or pitavastatin;
(2) a pharmaceutical composition in which the HMG-CoA reductase inhibitor is pravastatin, lovastatin, simvastatin, fluvastatin, atorvastatin, rosuvastatin or pitavastatin;
(3) a pharmaceutical composition in which the HMG-CoA reductase inhibitor is pravastatin, pitavastatin or atorvastatin; or
(4) a pharmaceutical composition in which the HMG-CoA reductase inhibitor is pravastatin or atorvastatin; or
(5) a pharmaceutical composition in which the HMG-CoA reductase inhibitor is pravastatin; or
(6) a pharmaceutical composition in which the HMG-CoA reductase inhibitor is atorvastatin; or
   more preferably includes:
(7) a pharmaceutical composition for administering N-(1-octyl-5-carboxymethyl-4,6-dimethylindolin-7-yl)-2,2-dimethylpropanamide sulfate and an HMG-CoA reductase inhibitor, or
(8) a pharmaceutical composition described in (7) in which the HMG-CoA reductase inhibitor is pravastatin, lovastatin, simvastatin, fluvastatin, rivastatin, pitavastatin, atorvastatin or rosuvastatin;
(9) a pharmaceutical composition described in (7) in which the HMG-CoA reductase inhibitor is pravastatin, lovastatin, simvastatin, fluvastatin, pitavastatin, atorvastatin or rosuvastatin; or
(10) a pharmaceutical composition described in (7) in which the HMG-CoA reductase inhibitor is pravastatin, atorvastatin or pitavastatin;
(11) a pharmaceutical composition described in (7) in which the HMG-CoA reductase inhibitor is pravastatin or atorvastatin;
   and particularly preferably includes:
(12) a pharmaceutical composition described in (7) for administering N-(1-octyl-5-carboxymethyl-4,6-dimethylindolin-7-yl)-2,2-dimethylpropanamide sulfate and atorvastatin, or
(13) a pharmaceutical composition described in (7) for administering N-(1-octyl-5-carboxymethyl-4,6-dimethylindolin-7-yl)-2,2-dimethylpropanamide sulfate and pravastatin.

### Effects of the invention

Since the pharmaceutical composition for administering simultaneously, separately or sequentially N-(1-octyl-5-carboxymethyl-4,6-dimethylindolin-7-yl)-2,2-dimethylpropanamide or a pharmacologically acceptable salt thereof and an HMG-CoA reductase inhibitor of the present invention have excellent lipid lowering action, have excellent progress inhibitory effects on atherosclerosis in the aorta, have excellent onset inhibitory effects on xanthoma occurring in limb joints, and have low toxicity, they are useful as a preventive or therapeutic agent (particularly a therapeutic agent) for atherosclerosis or xanthoma (particularly atherosclerosis) for warm-blooded animals (particularly humans).

### Industrial applicability

The N-(1-octyl-5-carboxymethyl-4,6-dimethylindolin-7-yl)-2,2-dimethylpropanamide or a pharmacologically acceptable salt thereof, which is an active ingredient of the pharmaceutical composition of the present invention, can be easily produced according to the method described in WO 97/12860 (EP 0866059) or the corresponding USP 6,063,806.

In addition, the HMG-CoA reductase inhibitor, which is an active ingredient of the pharmaceutical composition of the present invention, can be easily produced according to the method described in Japanese Patent Application (Kokai) No. Sho 57-2240 (USP 4,346,227), Japanese Patent Application (Kokai) No. Sho 57-163374 (USP 4,231,938), Japanese Patent Application (Kokai) No. Sho 56-122375 (USP 4,444,784), Japanese Patent Application (Kohyo) No. Sho 60-500015 (USP 4,739,073), Japanese Patent Application (Kokai) No. Hei 1-216974 (USP 5,006,530), Japanese Patent Application (Kokai) No. Hei 3-58967 (USP 5,273,995), Japanese Patent Application (Kokai) No. Hei 1-279866 (USP 5,854,259 and USP 5,856,336), or Japanese Patent Application (Kokai) No. Hei 5-178841 (USP 5, 260, 440).

Since the pharmaceutical composition for administering simultaneously, separately or sequentially N-(1-octyl-5-carboxymethyl-4,6-dimethylindolin-7-yl)-2,2-dimethylpropanamide or a pharmacologically acceptable salt thereof and an HMG-CoA reductase inhibitor of the present invention have excellent lipid lowering action, have excellent progress inhibitory effects on atherosclerosis in the aorta, have excellent onset inhibitory effects on xanthoma occurring in limb joints, and have low toxicity, they are useful as a preventive or therapeutic agent (particularly a therapeutic agent) for atherosclerosis or xanthoma (particularly atherosclerosis).

These combinations (the N-(1-octyl-5-carboxymethyl-4,6-dimethylindolin-7-yl)-2,2-dimethylpropanamide or a pharmacologically acceptable salt thereof and an HMG-CoA reductase inhibitor) provide a higher level of effectiveness and provide results unattainable by administration of the individual HMG-CoA reductase inhibitor or the dimethylpropanamide derivative alone.

The N-(1-octyl-5-carboxymethyl-4,6-dimethylindolin-7-yl)-2,2-dimethylpropanamide or a pharmacologically acceptable salt thereof and the HMG-CoA reductase inhibitor, which are the active ingredients of the pharmaceutical composition of the present invention, can be prepared in a separate pharmaceutical formulation in order to administer an HMG-CoA reductase inhibitor and the dimethylpropanamide derivative separately or in a single pharmaceutical formulation containing a mixture of an HMG-CoA reductase inhibitor and the dimethylpropanamide derivative in order to administer them at the same time.

In the case of using the pharmaceutical composition of the present invention as a preventive or therapeutic agent for the above diseases, the N-(1-octyl-5-carboxymethyl-4,6-dimethylindolin-7-yl)-2,2-dimethylpropanamide or a pharmacologically acceptable salt thereof and the HMG-CoA reductase inhibitor, which are the active ingredients of the pharmaceutical composition of the present invention, can be administered alone or after mixing with a pharmacologically acceptable vehicle or diluent and so forth, can be administered either orally in the form of tablets, capsules, granules, powders or syrup and so forth, or parenterally in the form of an injection or suppositories and so forth.

These preparations can be produced by well known methods using additives such as excipients (for example, organic excipients including sugar derivatives such as lactose, sucrose, glucose, mannitol and sorbitol; starch derivatives such as corn starch, potato starch, α-starch and dextrin; cellulose derivatives such as crystalline cellulose; gum arabic; dextran; and Pullulan; and inorganic excipients including silicate derivatives such as light silicic acid anhydride, synthesized aluminum silicate, calcium silicate and magnesium aluminate metasilicate; phosphates such as calcium hydrogen phosphate; carbonates such as calcium carbonate; and sulfates such as calcium sulfate), lubricants (for example, stearic acid and stearic acid metal salts such as calcium stearate and magnesium stearate; talc; colloidal silica; waxes such as beeswax and spermaceti wax; boric acid; adipic acid; sulfates such as sodium sulfate; glycol; fumaric acid; sodium benzoate; DL leucine; laurylsulfates such as sodium laurylsulfate and magnesium laurylsulfate; silicic acid derivatives such as silicic acid anhydride and silicic acid hydrate; and the above starch derivatives), binders (for example, hydroxypropyl cellulose, hydroxypropylmethyl cellulose, polyvinylpyrrolidone, macrogol, the above excipients and similar compounds), disintegrating agents (for examples, cellulose derivatives such as low-substituted hydroxypropyl cellulose, carboxymethyl cellulose, calcium carboxymethyl cellulose and internally bridged sodium carboxymethyl cellulose; and chemically-modified starches and celluloses such as carboxymethyl starch, sodium carboxymethyl starch and internally bridged polyvinylpyrrolidone), emulsifiers (for example, colloidal clay such as bentonite and veegum; metal hydroxides such as magnesium hydroxide and aluminum hydroxide; anionic surface active agents such as sodium laurylsulfate and calcium stearate; cationic surface active agents such as benzalkonium chloride; and nonionic surface active agents such as polyoxyethylene alkyl ether, polyoxyethylene sorbitan fatty acid ester and sucrose fatty acid ester), stabilizers (for example, parahydroxybenzoate esters such as methyl p-hydroxybenzoate and propyl p-hydroxybenzoate; alcohols such as chlorobutanol, benzyl alcohol and phenylethyl alcohol; benzalkonium chloride; phenols such as phenol and cresol; thimerosal; dehydroacetic acid; and sorbic acid), corrigents (for example, normally used sweeteners, souring agents and flavors), and diluents.

The dosage and administration ratio of the N-(1-octyl-5-carboxymethyl-4,6-dimethylindolin-7-yl)-2,2-dimethylpropanamide or a pharmacologically acceptable salt thereof and the HMG-CoA reductase inhibitor can be changed according to the activity of each active compound and symptoms, age, body weight and various other conditions of the patient.

Although the dose varies according to symptoms, age and so forth, the respective doses are a lower limit of 0.1 mg (preferably 0.5 mg) and an upper limit of 1000 mg (preferably 500 mg) per unit dose in the case of oral administration, and a lower limit of 0.01 mg (preferably 0.05 mg) and an upper limit of 100 mg (preferably 50 mg) per administration in the case of parenteral administration for adults (e.g. Human) one to six times per day, and administration can be performed simultaneously, separately or sequentially according to symptoms.

It should be noted that, in the case of applying the present invention to prevention or treatment of atherosclerosis, the dose of the HMG-CoA reductase inhibitor can be lowered to a lower dose than its dose when used for its known application as an antihyperlipemia agent. In addition, the dose can be lowered further due to the excellent effects resulting from the combined effects with N-(1-octyl-5-carboxymethyl-4,6-dimethylindolin-7-yl)-2,2-dimethylpropanamide or a pharmacologically acceptable salt thereof.

In addition, although the ratio of the doses of the N-(1-octyl-5-carboxymethyl-4,6-dimethylindolin-7-yl)-2,2-dimethylpropanamide or a pharmacologically acceptable salt thereof and the HMG-CoA reductase inhibitor, the active ingredients of the pharmaceutical composition of the present invention, can be varied over a wide range, for example, the ratio of the doses of N-(1-octyl-5-carboxymethyl-4,6-dimethylindolin-7-yl)-2,2-dimethylpropanamide or a pharmacologically acceptable salt thereof and the HMG-CoA reductase inhibitor can be administered over a range of 1:500 to 500:1 more usually 1:100 to 100:1, more preferably 1:10 to 10:1 and most preferably 1:5 to 5:1. When N-(1 -octyl-5-carboxymethyl-4,6-dimethylindolin-7-yl)-2,2-dimethylpropanamide is used with pravastatin as the HMG-CoA reductase inhibitor, it is preferable to use a ratio of 1:2.5 to 2.5:1; when it is used with atorvastatin as the HMG-CoA reductase inhibitor, it is preferable to use a ratio of 1:2.5 to 5:1. (All ratios are by weight.)

### Best mode for carrying out the invention

The present invention will be described below in more detail referring to its test example and preparation example, but the scope of the present invention is not limited to them.

### Test Example 1

### Lipid Lowering Action

Eleven-week-old male Fib hamsters were used in the experiment. The animals were fed in metal cages and allowed free access to food, which contained 0.05% cholesterol and 10% coconut oil, and water. The active agents were administered orally in the form of a suspension in 5% gum arabic solution once a day for 7 days. The animals were fasted for 17 hours after the final administration of active agents at which time blood samples were collected under anesthesia. Serum lipids were measured by an enzyme method. Those results are shown in Table 1. In the table, TC indicates total cholesterol, VLDL indicates very low density lipoprotein, LDL indicates low density lipoprotein, HDL indicates high density lipoprotein and compound A indicates N-(1-octyl-5-carboxymethyl-4,6-dimethylindolin-7-yl)-2,2-dimethylpropanamide sulfate.

**[Table 1]**

| | Cholesterol concentration | | | Atherogenic index |
|---|---|---|---|---|
| | TC | VLDL+LDL | HDL | |
| Control | 253.0 | 189.4 | 63.6 | 3.0 |
| Pravastatin (3 mg/kg) | 244.0 | 170.6 | 73.3 | 2.3 |
| Compound A (30 mg/kg) | 170.9 | 109.0 | 62.0 | 1.8 |
| Compound A (30 mg/kg) + Pravastatin (3 mg/kg) | 151.1 | 86.9 | 64.2 | 1.4 |

While administration of pravastatin or N-(1-octyl-5-carboxymethyl-4,6-dimethylindolin-7-yl)-2,2-dimethylpropanamide sulfate alone respectively decreased total serum cholesterol and serum VLDL+LDL cholesterol levels, when both active agents were used in combination, those effects were further enhanced. In addition, while administration of pravastatin or N-(1-octyl-5-carboxymethyl-4,6-dimethylindolin-7-yl)-2,2-dimethylpropanamide sulfate alone lowered the atherogenic index (VLDL+LDL cholesterol/HDL cholesterol), an indicator of susceptibility to atherosclerosis, those effects were further enhanced when the two active agents were used in combination.

### Test Example 2

### The Effect of the Development of Atherosclerotic Lesion in Cholesterol-Fed Rabbits

Male New Zealand White rabbits (Kitayama Labes, Japan) weighing 1.0-1.5 kg were meal-fed a chow diet (RC-4, Oriental Bioservice, Japan) supplemented with 0.5% cholesterol, 3% peanut oil, and 3% coconut oil diet for a total of 10 weeks.

The dietary regimen consisted of feeding the cholesterol/fat diet at 40 g for the first 2 weeks, 50 g for 4 weeks, 60 g for final 4 weeks. After 2 weeks of diet initiation, a chronic endothelial injury was induced in the abdominal aorta and right femoral artery by surgically inserting a sterile, nylon monofilament through the medical saphenous artery of the right leg to the level of the diaphragm. The monofilament was secured and maintained in place for remaining 8 weeks of the study. Animals were randomized on the basis of their total cholesterol levels after 2 week of cholesterol/fat diet. The HMG-CoA reductase inhibitor and the ACAT inhibitor were orally administered both alone and in combination for 8 weeks after surgery.

### (i) Atherosclerotic area

Femoral artery was photographed for the morphometric analyses by digital camera (Coolpix 990, trade name: product of Canon, Inc.) connected with a computer. The severity of aortic atherosclerosis was estimated as a ratio of surface area of lesion to the intimal surface area of aorta by Photo-retouch software ("Adobe Photoshop 5.0" trade name: product of Adobe Systems Inc.) and the Scion image NIH Imaging Software ("Scion Image 1.61c MacOs" product of Scion Corporation).

### (ii) Extracellular lipid deposits

Rabbits were anesthetized with an intravenous injection of sodium pentobarbital (25 mg/kg) and perfused with saline by using a perfusion apparatus at a constant pressure of 100 mmHg. After perfusion, the aortas were excised and photographed. Analysis of lesional cross-sections on aortas was examined at the right femoral artery. Right femoral artery was fixed in Methanol Carnoy's fixative for at least 24 hours. The segments were embedded in paraffin, and sectioned at 5 µm. Five ribbons of four serilal sections from each segment were cut at 80 µm intervals. A section from each ribbon was treated with Elastica-Masson's stain.

A digital image of each section was collected using a digital camera (Fujix CCD camera system HC-2500: product of Fuji Photo Film Co., Ltd.) attached to a Leica microscope at a magnification of 2.5 x. The area of each lesional component was extracted using Adobe Photoshop 5.0 (Adobe) for estimation of the quality of the lesions. In sections treated with Elastica-Masson's stain, the region with light green were identified as extracellular matrix (ECM), and extracellular vacuoles and lacunae (white regions) were defined as extracellular lipid deposits (ECD). ECD was measured by average of value dividing area of lesional component by area of lesion using Adobe Photoshop 5.0 and Scion Image 1.61 MacOs.

The results are shown in Table 2. In the table, compound A indicates N-(1-octyl-5-carboxymethyl-4,6-dimethylmdolin-7-yl)-2,2-dimethylpropanamide sulfate.

**[Table 2]**

| | Ratio of atherosclerotic area (%) | ECD (mm²) |
|---|---|---|
| Control | 57.4 | 15.5 |
| Pravastatin (10 mg/kg) | 58.3 | 16.6 |
| Compound A (5 mg/kg) | 45.8 | 10.7 |
| Compound A (5 mg/kg) + Pravastatin (10 mg/kg) | 40.9 | 3.0 |

As shown by the results above, while compound A reduced the atherosclerotic area of the femoral lesion and ECD, and pravastatin had no effect on the area and ECD, when both active agents were used in combination, those effects were synergistically enhanced.

### Preparation Example 1

### Tablets

Pravastatin sodium salt (10.0 mg), N-(1-octyl-5-carboxymethyl-4,6-dimethylindolin-7-yl)-2,2-dimethylpropanamide sulfate (30.0 mg), lactose (408.0 mg), corn starch (50.0 mg) and magnesium stearate (2.0 mg) are mixed and formed into a tablet by a tablet making machine to obtain a tablet containing 500 mg. The tablet can be coated (preferably with a sugar coating) as necessary.

## Claims

1. A pharmaceutical composition comprising administering simultaneously, separately or sequentially N-(1-octyl-5-carboxymethyl-4,6-dimethylindolin-7-yl)-2,2-dimethylpropanamide or a pharmacologically acceptable salt thereof, and an HMG-CoA reductase inhibitor.

2. The pharmaceutical composition according to claim 1 wherein the HMG-CoA reductase inhibitor is pravastatin, lovastatin, simvastatin, fluvastatin, rivastatin, atorvastatin, rosuvastatin or pitavastatin.

3. The pharmaceutical composition according to claim 1 wherein the HMG-CoA reductase inhibitor is pravastatin, lovastatin, simvastatin, fluvastatin, atorvastatin, rosuvastatin or pitavastatin.

4. The pharmaceutical composition according to claim 1 wherein the HMG-CoA reductase inhibitor is pravastatin, atorvastatin or pitavastatin.

5. The pharmaceutical composition according to claim 1 wherein the HMG-CoA reductase inhibitor is pravastatin or atorvastatin.

6. The pharmaceutical composition according to claim 1 wherein the HMG-CoA reductase inhibitor is pravastatin.

7. The pharmaceutical composition according to claim 6 wherein the HMG-CoA reductase inhibitor is atorvastatin.

8. The pharmaceutical composition comprising administering simultaneously, separately or sequentially N-(1-octyl-5-carboxymethyl-4,6-dimethylindolin-7-yl)-2,2-dimethylpropanamide sulfate, and an HMG-CoA reductase inhibitor.

9. The pharmaceutical composition according to claim 8 wherein the HMG-CoA reductase inhibitor is pravastatin, lovastatin, simvastatin, fluvastatin, rivastatin, atorvastatin, rosuvastatin or pitavastatin.

10. The pharmaceutical composition according to claim 8 wherein the HMG-CoA reductase inhibitor is pravastatin, lovastatin, simvastatin, fluvastatin, atorvastatin, rosuvastatin or pitavastatin.

11. The pharmaceutical composition according to claim 8 wherein the HMG-CoA reductase inhibitor is pravastatin, atorvastatin or pitavastatin.

12. The pharmaceutical composition according to claim 8 wherein the HMG-CoA reductase inhibitor is pravastatin or atorvastatin.

13. The pharmaceutical composition according to claim 8 comprising administering simultaneously, separately or sequentially N-(1-octyl-5-carboxymethyl-4,6-dimethylindolin-7-yl)-2,2-dimethylpropanamide sulfate and atorvastatin.

14. The pharmaceutical composition according to claim 8 comprising administering simultaneously, separately or sequentially N-(1-octyl-5-carboxymethyl-4,6-dimethylindolin-7-yl)-2,2-dimethylpropanamide sulfate and pravastatin.
